# EUROPEAN PATENT APPLICATION

(11) **EP 1 433 374 A1**
(43) Date of publication of application: **30.06.2004**
(21) Application number: 03029954.9
(22) Date of filing: 29.12.2003
(51) Int. Cl.: A01G 1/04

(54) **Method for incubating Pleurotus nebrodensis and disease preventing/treating agents from Pleurotus nebrodensis**

(30) Priority: 26.12.2002 JP 2002378014; 26.05.2003 JP 2003147895
(71) Applicant: Kitajima, Yoshinobu, Mizuma-gun, Fukuoka 830-0415 (JP); Eguchi, Fumio, Takasaki-shi, Gunma 370-1201 (JP); Kitamoto, Yutaka, Tottori-shi, Tottori 680-0862 (JP)
(72) Inventor: Kitajima, Yoshinobu, Mizuma-gun, Fukuoka 830-0415 (JP); Eguchi, Fumio, Takasaki-shi, Gunma 370-1201 (JP); Kitamoto, Yutaka, Tottori-shi, Tottori 680-0862 (JP)
(74) Representative: Henkel, Feiler & Hänzel

(57) **Abstract**

The objective of this invention is to provide a method for incubating *Pleurotus nebrodensis* in a way that yields a sufficient amount of fruiting bodies within a short period and to offer a disease preventing/treating agent made from *Pleurotus nebrodensis*.

Using the incubation method listed in this invention, fruiting bodies can be obtained at a sufficient yield within a short period by the means of administering an electric impulse and/or a temperature change.

Also a disease preventing/treating agent in *Pleurotus nebrodensis* in this invention exhibits an excellent disease preventing/treating effect.

## Description

### RELATED APPLICATIONS

This application claims priority to Japanese Patent Applications Nos. 2002-378014 filed December 26, 2002, and Nos. 2003-147895 filed May 26, 2003, which are hereby incorporated with references for all purposes.

### FIELD OF THE INVENTION

This invention relates to a method for incubating and increasing the yield of *Pleurotus nebrodensis (P. nebrodensis)* and the discovery of an agent from *P. nebrodensis* with a disease preventing/treatment effect.

### BACKGROUND OF THE INVENTION

Recently, due to a larger variety in diets and an increased popularity in refined foods, there is a rise in obesity, even among younger generations. Also, with an aging population and more stressful lifestyles, cases of age and lifestyle diseases are predicted to be more widespread. Hypertension, especially, is expected to be on the rise due to increased obesity and lack of exercise. In addition, hypertension has been linked frequently to diseases such as diabetes, sugar intolerance, hyperlibedimia, lipid dysbolism, obesity and the like.

Conventionally, Ca++ antagonists, β receptor antagonists, angiotensin converting enzyme inhibitors, etc. are known to be effective and are employed widely as agents for treating hypertension. However, a prolonged period of treatment with such agents has a risk of causing problematic side effects. The main treatments for obesity, such as exercise therapy and dietetic therapy are hard to sustain. Treatment using natural or health foods (including functional foods), which are capable of being ingested continuously without much risk of side effects, are products with a great interest these days.

The demands for edible mushrooms are increasing due to more diverse eating habits and the mushroom's status as a health food, and so their yield is also increasing. Incubating technology has been developed corresponding to each mushroom type. Except for some mushrooms that have unusual biological characteristics, almost all mushrooms such as *Lentinus edodes, Flammulina velutipes, Hypsizygus marmoreus, Grifola frondosa, Plerotus ostreatus, Pholiota nameko, Pleurotus eryngii* are subjected to artificial incubation.

Examples of different types of incubation include using raw lumber for *Lentinus edodes* and in-pot fungal bed incubation for *Flammulina velutipes, Pleurotus ostreatus* which uses a culture medium consisting of a mixture of sawdust, rice bran, wheat bran, water, etc. In in-pot fungal bed incubation, various supplemental materials such as red algae powder (JP-A-06-113670) and ground eggshells (JP-A-06-253677) are regularly used for the purpose of increasing mushroom yield and/or improving quality. The composition of the culture medium has a substantial effect on the growth of the mushroom.

However, in the case of *P. nebrodensis*; it is difficult to obtain sufficient production yield using conventional incubation methods, and so there is a great demand for an incubation method capable of increasing yield.

### SUMMARY OF THE INVENTION

This invention is established on the basis of the problems addressed above and its objective is to provide a method for incubating *P. nebrodensis* that is capable of yielding a sufficient amount of fruiting bodies within a short period and supplement containing the disease preventing/treating agents from *P. nebrodensis*.

In order to reach the objective described above, the inventors had discovered that fruiting bodies of the *P. nebrodensis* are induced when treated with an electrical impulse and/or temperature change. It has also been discovered that *P. nebrodensis* possesses disease preventing/treating agents, whereby establishing this invention.

The first aspect of this invention is a method for incubating P. *nebrodensis*. Conventionally, the incubation of *P. nebrodensis* is characterized by an inoculation and cultivation of *P. nebrodensis* in a culture medium allowing mycelium to proliferate over the medium and generate fruiting bodies in which low temperature is maintained uniformly in a former-generating stage and increased in a latter-generating stage. For this method, temperature should be set at -5 to +3°C for the former-generating stage and 14-22°C for the latter-generating stage and the temperature raised in two steps. Humidity is maintained at 75-85% then increased to 90-100% when the temperature is raised. With the increase in temperature, the carbon dioxide level and/or illumination light intensity is to be increased. Before increasing the temperature, during the generating stage, a dead bacterial layer in the medium is also to be removed.

In this invention, the method is characterized by an inoculation and cultivation of a *P. nebrodensis* inoculum in a culture medium that allows mycelium to proliferate over the culture medium and generate fruiting bodies. This first stage has the surrounding temperature maintained uniformly in the early cultivation stage. Temperature then is decreased in the mid-cultivation stage and sharply increased in the late cultivation stage. After the cultivating state, an electric impulse between 5 and 60 kV is to be given. The temperatures during the three cultivation stages should be 16-24°C during the early-cultivation stage, 6-14°C during the mid-cultivation stage and 26-34°C during the late cultivating stage. The duration of the early-cultivation stage should be between 35 and 45 days, the mid-cultivation stage 5 to 15 days and the late-cultivation stage 5 to 15 days. Humidity should be maintained between 65 and 75% during the cultivating stages.

The second aspect of this invention is a method for incubating *P. nebrodensis* comprising of steps (a) through (d):
(a) A step for inoculating *P. nebrodensis* in a culture medium
(b) A step for incubation at a temperature of 20 to 30°C whereby allowing the mycelium to proliferate over the culture medium after step (a);
(c) A step for when electric impulse is given at a voltage between 5 and 60 kV after step (b);
(d) A step fro generating fruiting bodies at a temperature of 10 to 20°C after step (c).

In the method for incubating described above, in step (d), the temperature should be temporarily decreased at -1 to 2°C and then increased to 10 to 20°C.

The third aspect of this invention is a disease preventing/treating agent in which *P. nebrodensis* is the active ingredient. The agent is preferably comprised of dried *P. nebrodensis* powder and/or a hot water extraction. The diseases that the agent is to prevent/treat is one or more of the following: hypertension, hyperlipidemia and obesity.

### BRIEF DESCRIPTION OF THE DRAWINGS

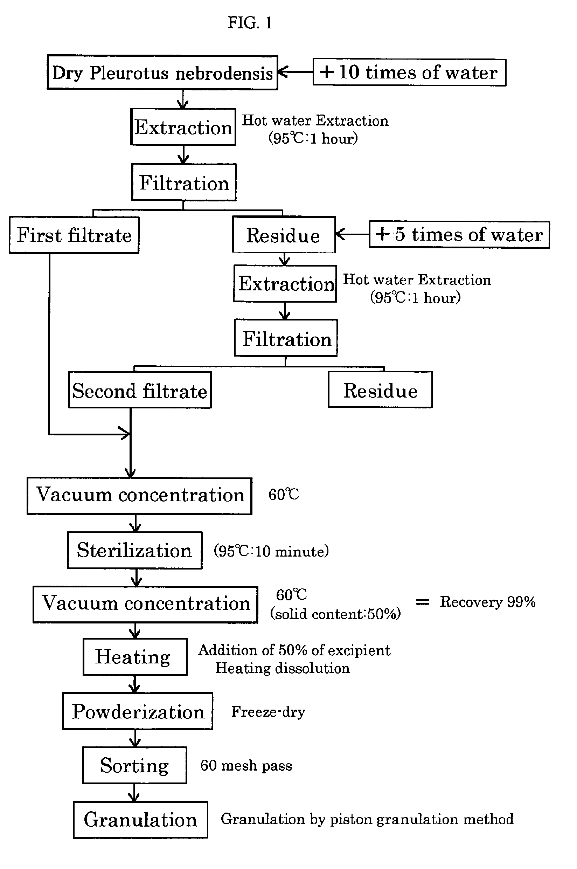

Figure 1 shows a flowchart of a method for preparing a test substance according to this invention.

### DETAILED DESCRIPTION OF THE PREFERRED EMBODIMENT

Preferred embodiments of this invention this invention, a material serving as a culture medium is not limited particularly as long as the effect of this invention is not prevented and may be material employed conventionally. For example, a formulation obtained by mixing rice bran, sawdust and water may be employed. It may contain are described below.

First is the method for incubating *P. nebrodensis* according to this invention:

### (1) Preparation of the Culture Medium

In this invention, a wide range of culture medium ingredients may be used as long as the effect of this invention is not compromised. For example, standard culture material, such as a mixture of rice bran, sawdust and water may be used. The culture also can contain mycelium activating agents and such. ,

Any suitable container for the culture medium can be used as long as it does not interfere with the effectiveness of this invention. Usually a culture bottle, a bottle-shaped container with an opening at the top or a culture bag, a bag-like container, are used.

### (2) Sterilization of the Medium

A culture medium is to be sterilized by some sort of sterilization method such as an autoclave. Standard conditions for sterilization are 100°C for about 4 hours, but there are no limitations on sterilizing conditions.

### (3) Inoculation

When the culture medium is cooled to about 20°C a suitable hole is to be made in the medium approximately at the center of the container containing the medium and inoculated with an inoculation of P. *nebrodensis.* The method for the inoculation is not limited particularly as long as the effect of this invention is not compromised and any ordinary method may be used. While the depth of the hole does not have to be at a certain length, it is preferable that the depth reaches a level close to the bottom of the container so that the *P. nebrodensis* microorganism may be well distributed.

### (4) Cultivation

After the inoculation, the mycelium should be allowed to proliferate over the culture medium. The conditions during this stage should be at a room temperature between 20 and 30°C and humidity at 70 to 100% and be kept as such until there is a sufficient proliferation of mycelium. While the conditions of this stage are not limited as long as it is in the constraints listed above, it is recommended that for the purpose of rapid proliferation, that the room temperature is lowered transiently during the middle stage of this incubation period and then elevated again.

Typically, the room temperature at an early-cultivating stage is around 20°C (between 16 and 24°C). The transiently lowered room temperature during the mid-cultivating stage is 10°C (6 to 14°C) and the elevated temperature at the late cultivating stage is 30°C (26 to 34°C).

The duration of the early-cultivating stage is preferably about 40 days, while the length of the mid and late-cultivating stages are about 10 days. The humidity should be maintained around 70% (65 to 75%).

### (5) Generation of Fruiting Bodies

After the mycelium has proliferated over the culture medium, it is to be treated with an electric impulse to promote the generation of fruiting bodies and also to increase the yield. The electric impulse should be given somewhere between 5 and 60kV, preferably between 20 and 30kV and optimally at 20kV. An impulse less than 5 kV may lead to an inefficient result in this invention, while an impulse exceeding 60 kV may lead to difficulty in the generation of the fruiting body. The time point at which the electric impulse is given is preferably the time when the fruiting body has started to generate after cultivation.

In order to allow a fruiting body to be generated, the room temperature should be reduced to a level lower than that upon cultivation and should preferably between 10 and 20°C with humidity of 80 to 100%.

While the conditions of the temperature and the humidity suitable to the generation of the fruiting body are specified above, the room temperature should preferably kept at a low temperature and thereafter elevated for the purpose of promoting the generation of the fruiting body to increase yield. Typically, the mycelium is exposed to a low temperature by keeping the temperature at around -1°C (-5°C to +3°C) for about 5 days, followed by 5 days kept around 5°C (1 to 9°), followed by an elevated temperature at around 18°C (14 to 22°C). During this period, the temperature may be adjusted appropriately depending on the condition of the mycelium.

It is preferable that the humidity is elevated from around 80% (75-85%) to around 95% (90-100%) almost simultaneously at the rise of temperature. The layer containing dead bacteria at the surface of the culture medium should also be removed. The layer removed should be around 15mm deep.

With the rise of temperature, the carbon dioxide level and illumination light intensity must also be raised. The carbon dioxide level should be elevated from about 400ppm to around 2000 ppm. The illumination light should be blue and its light intensity should be increased from about 100 Lux to about 400 Lux.

By using *P. nebrodensis* grown according to the methods described in this invention, a disease preventing/treating agent can be produced from *P. nebrodensis.* These agents, when created by methods specified in this invention, can be given orally and be used to in prevention and treatment of certain diseases, particularly hypertension, hyperlipidemia and obesity. The dose of the agent is not specified and may vary depending on the condition. The efficacy of the agent has been seen in adults given 1 to 15 g of dried *P. nebrodensis* a day. Especially when given a dose between 6 and 9g one can expect a certain effect.

*P. nebrodensis* can also be ingested as an extract from dried powder form. Such an extract can be made by adding the dried powder of the fruiting body of *P. nebrodensis* to hot water. The extract may be administered as a liquid or as a dried extract. This disease preventing/treating agent may also be administered as an oral formulation. When given as an oral formulation, the agent may be form of a powder, tablet, capsule, granule, tea, suspension, liquid extract, liquid, syrup, etc. Upon formulation, an ordinary carrier, such as an excipient, binder, disintegrant, lubricant, colorant, flavor, fragrance, etc. may be employed if necessary. A suitable coating may also be employed to form a film-coated formulation.

This invention is further described in the examples shown below which are not intended to restrict this invention.

A culture medium consisting of 150 parts by weight (pbw) of sawdust, 100 pbw of wheat bran, 10 pbw of corn meal and 15 pbw of a mycelium activator were combined and water content adjusted to about 65%. About 650g of the prepared culture medium were added to polypropylene culture bottles (opening: 52 mm in diameter, volume: 850 mL) and the bottles sealed.

The culture medium was then autoclaved at 121°C for 4 hours and cooled to a temperature of 20°C or less. A hole of 10mm in diameter was opened in the center of the culture medium until it reached the bottom of the bottle and 15g of *P. nebrodensis* inoculants were poured into this hole.

The bottle inoculated with the inoculants was placed in a cultivation room whose room temperature and relative humidity were set at 21°C and 70% for 40 days, then at 10°C and 70% for 10 days and finally at 30°C and 70% for 10 days. As a result, the mycelium had proliferated sufficiently over the medium culture and reached maturation. The bottle was then transferred to a room whose temperature and relative humidity were set at -1°C and 80% for several days, then at 5°C and 80% for 7 days and then the surface of the bacterial floor was removed (depth: 15mm and diameter 35mm)(Bacteria scratch).

Then, the room temperature and relative humidity of the growth room were set at 18°C and 95%, the carbon dioxide level was increased from 400 ppm to 2000 ppm and the illumination intensity of a blue fluorescence lamp was raised from 100 Lx to 400 Lx.

As a result, fruiting bodies were gathered at about 210g per culture bottle at the 21^{st} day from bacteria scratch.

Furthermore, at the end of incubation, an electric impulse between 5 to 60 kV, specifically 15 to 30 kV was given which resulted in a higher yield of fruiting bodies in a shorter period of time. Accordingly, it was revealed that it is preferable to give an electric impulse in addition to the temperature change.

### Effect of Electric Impulse

1. A culture medium consisting of 150 pbw sawdust, 100 pbw wheat bran, 10 pbw corn meal and 15 pbw mycelium activator with water content adjusted to around 65% was provided. About 650g of the culture medium was and added to polypropylene culture bottles (opening: 52 mm in diameter, volume: 850 mL) and the bottles were sealed.
2. The culture medium was autoclaved at 121° for 4 hours and cooled to a temperature not higher than 20°C.
3. The center of the culture medium was opened with a hole about 20mm in diameter and reached to the bottom of the bottle. The hole then received 15g of *P. nebrodensis* inoculants.
4. The bottles inoculated with *P. nebrodensis* were placed for 60 days in a cultivation room whose room temperature and relative humidity were set at 25°C and 70%, respectively. As a result, the mycelium proliferated sufficiently over the medium and matured.
5. The bottles thus cultured, were treated with an electrical impulses at a predetermined voltage.
6. The bottles were then transferred to a growth room whose room temperature and relative humidity were set at 10°C and 90% and checked for generation of fruiting bodies every other day.

For each level of voltage, a 100 culture bottles were provided. The number of culture bottles displaying fruiting bodies on days of incubation along with the mean yield is shown in Table 1. The mean yield is the mean weight of all the fruiting bodies per culture bottle at each level of voltage. The numbers of incubation days were counted starting from the day of the electric impulse treatment (the day of the transfer from the cultivation room to the growth room).

**TABLE 1**

| Example | 1 | 2 | 3 | 4 | 5 | 6 | 7 |
|---|---|---|---|---|---|---|---|
| Voltage (kV) | 0 | 5 | 10 | 15 | 20 | 25 | 30 |
| The number of the culture bottles in which the fruiting bodies had generated. | | | | | | | |
| 14^{th} day | 0 | 0 | 0 | 0 | 1 | 3 | 8 |
| 16^{th} day | 0 | 0 | 2 | 4 | 9 | 14 | 24 |
| 18^{th} day | 0 | 3 | 7 | 8 | 17 | 28 | 39 |
| 20^{th} day | 0 | 11 | 19 | 32 | 26 | 25 | 29 |
| 22^{nd} day | 0 | 23 | 34 | 29 | 23 | 30 | 0 |
| 24^{th} day | 0 | 25 | 19 | 25 | 24 | 0 | 0 |
| 26^{th} day | 1 | 14 | 7 | 2 | 0 | 0 | 0 |
| Total | 1 | 76 | 88 | 100 | 100 | 100 | 100 |
| Mean Yield (g) | 73 | 94 | 107 | 135 | 172 | 164 | 159 |

As evident from Table 1, the electric impulse treatment resulted in earlier generation of fruiting bodies which led to increased yield. The earliest date of the fruiting body generation was earlier at higher voltages. The fruiting body began to generate on the 14^{th} day at 20 to 30 kV, on the 16^{th} day at 10 to 15 kV and on the 18^{th} day at 5 kV. All culture bottles exhibited the generation of fruiting bodies no later than the 26^{th} day at 15 kV, the 22^{nd} day at 25 kV and the 20^{th} day at 30 kV.

The highest yield was observed at 20kV and the yield at 20 to 30 kV was double of that without the electric pulse treatment. A test at even higher voltages revealed that the day of fruiting body generation at 60 kV exhibiting a yield increasing effect.

Thus it has been revealed that in order to obtain a high yield of *P. nebrodensis* within a short period, it is advantageous to give an electric impulse at the end of the incubation at a voltage between 5 to 60 kV, specifically between 15 and 30 kV, with 20 kV being the most preferred voltage level in terms of yield.

The temperature conditions stated below were also used resulting in an even higher yield. In addition, it was discovered that it is advantageous to change the temperature in addition to dispensing electric impulse.

### Temperature Conditions:

The room temperature and the relative humidity were kept at -1°C and 90% for 5 days. Room temperature then was elevated to 5°C for 5 days and elevated again to 18°C.

### 1.Disease Preventing/Treating Agents Against Hypertension

### (1) Methods for Breeding Test Rats and for Administering Test Substances

### [1] Preliminary Breeding (6 to 7 weeks old)

6-week old male spontaneous hypertensive rats (SHR) and normotensive Wistar Kyoto Rats (WKY) purchases from Charles River Japan Co. Inc were maintained in an animal chamber kept at room temperature of 22±1°C and relative humidity of 60±10% under the light/dark cycle of 12 hours a day (lighting from 7:00 to 9:00) using white fluorescent lamps. The rats were allowed free feeding of food and tap water for 1 week during the preliminary breeding period.

After the preliminary breeding, the individual animals were weighed and examined. Blood pressure was taken and the rats were split into groups of eight based on rat type and so that the mean blood pressure of the animals were basically consistent between the groups. The SHR rats were split into groups A through H and the WKY rats were split into groups I to J.

### [2] Preparation of P. nebrodensis Extract

The powder used for extract was made by grinding dried fruiting body of *P. nebrodensis* was ground and straining through a 16 mesh sieve. Extract was made by seeping the at 3, 6, 9 and 12 grams of the powder in 600 mL of hot water for 2 hours. Hereinafter, the extracts were referred as "3g Extract", "6g Extract" "9g Extract" and "12g Extract".

### [3] Administration of Test Substance (at 8 to 20 weeks old)

In accordance with the procedure stated below, the rats in each group were treated with the test substances for 12 weeks, from when they were 8 weeks old to 20 weeks old. The animals were allowed to feed and drink tap water freely. The animal chambers were kept at a room temperature of 22±1°C and a relative humidity of 60±10% under light/dark cycles of 12 hours of light a day (lighting from 7:00 to 19:00) using a white fluorescence lamp.

The animals were treated orally with the extract at 10:00 am everyday followed by sterile water, which was given freely.
Group A: SHR Rats (no treatment)
Group B: SHR Rats (3 g Extract Treatment Group)- 10 mL of the 3g extract per kg of body weight were administered daily
Group C: SHR Rats (6 g Extract Treatment Group)- 10 mL of the 6g extract per kg of body weight were administered daily
Group D: SHR Rats (9 g Extract Treatment Group)- 10 mL of the 9g extract per kg of body weight were administered daily
Group E: SHR Rats (12 g Extract Treatment Group)- 10 mL of the 12g extract per kg of body weight were administered daily
Group F: SHR Rats (3% Dried Powder)- The feed was mixed with 3% *P. nebrodensis* dried powder and the rats were allowed to feed freely
Group G: SHR Rats (6% Dried Powder)- The feed was mixed with 6% *P. nebrodensis* dried powder and the rats were allowed to feed freely
Group H: SHR Rats (9% Dried Powder)- The feed was mixed with 9% *P. nebrodensis* dried powder and the rats were allowed to feed freely
Group I: WKY Rats (no treatment)
Group J: WKY Rats (6g Extract Treatment Group)- 10 mL of the 6g extract per kg of body weight were administered daily

### (2) Results

### [1] Blood Pressure Test

The blood pressure was taken once per 2 weeks before the administration of the test substance and the mean blood pressure of each group of rats was determined. Results are shown in Table 2.

**TABLE 2**

| Group | SHR Rats | | | | | | | | WKY Rats | |
|---|---|---|---|---|---|---|---|---|---|---|
| | A | B | C | D | E | F | G | H | I | J |
| 6 weeks old | 142 | 142 | 142 | 142 | 142 | 142 | 142 | 142 | 142 | 142 |
| 8 weeks old | 172 | 169 | 171 | 176 | 169 | 172 | 170 | 167 | 134 | 127 |
| 10 weeks old | 189 | 187 | 189 | 186 | 179 | 186 | 187 | 176 | 143 | 135 |
| 12 weeks old | 201 | 208 | 204 | 197 | 201 | 200 | 209 | 187 | 147 | 142 |
| 14 weeks old | 218 | 222 | 204 | 191 | 198 | 203 | 193 | 194 | 143 | 144 |
| 16 weeks old | 219 | 217 | 212 | 188 | 193 | 217 | 184 | 187 | 139 | 141 |
| 18 weeks old | 229 | 227 | 201 | 184 | 182 | 219 | 176 | 182 | 142 | 139 |
| 20 weeks old | 223 | 224 | 196 | 174 | 177 | 224 | 179 | 188 | 140 | 143 |

The Spontaneous Hypertensive Rats (SHR) groups A through H exhibited higher blood pressures when compare to the Wistar Kyoto Rats (WKY) groups I and J. However, in the SHR rats, an inhibition in blood pressure elevation was seen from the time of 14 weeks to 20 weeks in the 6, 9 and 12g treatment groups (Groups C, D and E) when compared to the group with no treatment, Group A. It was also noted that blood pressures actually dropped at 14 weeks for the 6% group (Group G), 16 weeks for the for the 9 and 12g and 9% groups (Groups D, E and H) and at 18 weeks for the 6 g group (Group C).

Higher doses led to greater hypertension inhibiting and hypertensive effects. The fact that the hypotesnsive effect in the 12 g group (Group E) was not higher than that of the 9g group (Group D) may be probably due to the expression of the biological curve in response to the efficacy dose. The naturally normotensive WKY rats exhibited no reduction in blood pressure.

As a result, it was seen that the *P. nebrodensis* according to this invention has a dose-dependent effect on the prevention and treatment of hypertension. The fact that in the normotensive cases (Groups I and J), no hypotensive effect was shown indicated that this invention may be able to be used safely since it seems to adjust the blood pressure to a normal level as opposed to conventional drugs used to treat hypertension, which reduce blood pressure regardless of whether the blood pressure is at a normal level or not. Also the powder or extract treatment displayed the same results.

### [2] Blood Test

The day prior to the final day of the experiment all rats were fasted. On the last day, the rats were heavily anesthetized with 45mg/kg, i.p. of Nembutal and blood was taken in a large as possible dose using a 20G needle on the left ventricle.

The blood sample was subjected to biochemical examination (automatic chemical analyzer: Auto Lab, Radio Immuno Assay method) and examined for the components listed in Table 3. The mean values of the level of the blood components were then calculated.

**TABLE 3**

| Group | SHR Rats | | | | | | | | WKY Rats | |
|---|---|---|---|---|---|---|---|---|---|---|
| | A | B | C | D | E | F | G | H | I | J |
| Total Cholesterol (mg/dL) | 45.4 | 50.1 | 54.2 | 54.7 | 56.8 | 54.1 | 56.1 | 56.8 | 60.5 | 61.4 |
| Free Cholesterol (mg/dL) | 6 | 5 | 6 | 7 | 7 | 6 | 6 | 7 | 7 | 7 |
| HDL Cholesterol (mg/dL) | 34 | 44 | 46 | 54 | 55 | 43 | 56 | 55 | 54 | 53 |
| LDL Cholesterol (mg/dL) | 10 | 7 | 8 | 6 | 5 | 6 | 5 | 5 | 5 | 5 |
| β Lipoprotein (mg/dL) | 38 | 34 | 32 | 30 | 28 | 28 | 33 | 32 | 28 | 29 |
| Triglycerides (mg/dL) | 43 | 36 | 32 | 30 | 31 | 34 | 33 | 31 | 34 | 32 |
| Total Protein (g/dL) | 6.2 | 6.2 | 6.4 | 7 | 7.4 | 6.6 | 6.9 | 7.2 | 7.1 | 7.2 |
| Albumin (g/dL) | 3.8 | 3.6 | 3.7 | 3.4 | 3.3 | 3.6 | 3.3 | 3.4 | 3.2 | 3.3 |
| A/G | 1.53 | 1.63 | 1.78 | 1.85 | 2.08 | 1.77 | 1.95 | 2.2 | 2.4 | 2.3 |
| White Blood Cell Count (10³/µL) | 3.9 | 4.2 | 5.3 | 5.4 | 5.6 | 5.2 | 5.8 | 5.9 | 6.1 | 6.3 |
| Urea Nitrogen (mg/dL) | 20 | 20.5 | 20.1 | 18.5 | 17.8 | 19.7 | 16.8 | 15.7 | 14.8 | 15 |
| Creatinine (mg/dL) | 0.6 | 0.58 | 0.61 | 0.53 | 0.53 | 0.59 | 0.54 | 0.55 | 0.52 | 0.54 |
| Uric Acid (mg/dL) | 3.1 | 2.7 | 2.6 | 2.2 | 2.3 | 2.6 | 2.4 | 2.4 | 2.3 | 2.2 |
| Renin | 2.8 | 2.5 | 2.6 | 2.7 | 2.3 | 2.6 | 2.4 | 2.3 | 3 | 3.1 |
| Angiotensin | 62.9 | 56.7 | 46.9 | 44.8 | 45.1 | 54.9 | 45.9 | 44.1 | 45.3 | 44.9 |

When compared with the WKY rats (Groups I and J), the SHR rats (Groups A through H) displayed higher values of LDL cholesterol, β lipoprotein, neutral fat, albumin, urea nitrogen, creatinine, uric acid and angiotensin. The SHR rats exhibited lower levels of total cholesterol, HDL cholesterol, total protein, A/G ratio and white blood cells. Nevertheless, the SHR rats exhibited a marked improvement in the levels of each component when compared to the control, the group with no treatment (Group A).

### Angiotensin

Angiotensin is an active peptide produced as a result of renin secretion produced by renal glomerulii on renin substrate in the liver. Because high levels of angiotensin are seen in cases of vasculogenic hypertension, malignant hypertension, etc., angiotensin levels serve as an index for hypertension.

When compared with Group A (the control), reduced levels of angiotensin were seen in Groups B through H. The levels were close to that of the normotensive Group I, especially in Groups B through E, G and H. Thus, besides reduced blood pressure, the effects of hypertension prevention/treatment were also seen in through angiotensin levels.

### Cholesterol

Cholesterol is an essential component of the body; for example, it is a constituent of the viable cell membrane. It is classified into two groups- HDL (good cholesterol) and LDL (bad cholesterol). LDL promotes arteriosclerosis, whereas HDL serves to remove LDL deposits from the walls of blood vessels. By measuring these cholesterol levels, lipid dysbolism can be diagnosed.

When compared with Group A, an increase in the HDL and a reduction in LDL were observed in SHR rat groups B through H. The values were close to the normotensive group I, especially groups D, E, G and H. Therefore, along with the effect of hypertension prevention/treatment, an effect of prevention or treatment for lipid dysbolism was also seen.

### β Lipoprotein and Neutral Fat

The major function of β lipoprotein is to transport cholesterol from the liver to each organ. Consequently, β lipoprotein levels serve as an index for lipid dysbolism associated diseases.

When compared with Group A, a dose-dependent reduction in the β lipoprotein level was noted in Groups B to H and the levels were closer to normal levels. Also, dose-dependent reductions in neutral fat levels were noted in Groups B through H. Thus, effects of lipid dysbolism prevention/treatment were seen through β lipoprotein and neutral fat levels.

### Urea Nitrogen, Creatinine and Uric Acid

The measured values of the urea nitrogen, creatinine and uric acid are employed as an index of hepatic and renal functions. When compared with Group A, the levels of urea nitrogen, creatinine and uric acids in Groups D, E, G and H were reduced and were closer to that in the normal group, Group I. The disease preventing/treating effect on hepatic and renal functions were seen in addition to the previously stated effects on hypertension.

### Total Protein, Albumin, A/G Ratio and White Blood Cell Count

Compared to the control (Group A) levels of total protein, A/G ratio and white blood cells were lowered and albumin levels rose in a dose-dependent manner in groups B through H. The levels were close to that of the healthy rats in group I.

Based on the findings described above the administration of *P. nebrodensis* was revealed to exert an effect of disease prevention and improvement regarding hypertension, lipid dysbolism, hepatic and renal functions.

In the normal rats Groups I and J, no abnormalities were seen in the values above, which indicate that the product may be safe to use.

### 2. Disease Prevention and Treatment Agent Against Hyperlipidemia and Obesity

The Zucker rat's genome is that of a simple recessive inheritance form and only an individual that possesses both recessive pathogenic genes (fa/fa), the Zucker-fatty rat {(ZUC)-fa/fa}, will exhibit obesity. Heterozygotes (fa/+) and wild types (+/+), Zucker-lean rats {(ZUC)-lean}, will not be obese. A Zucker-fatty rat will begin to exhibit signs of obesity at about 4 weeks of age. There will be some weight gain and an appearance of plumpness and the size of the rat will advance rapidly until about 10 weeks of age, followed by gradual progression of size. In addition, hyperlipidemia, hyperinsulinemia, hyperleptinemia and abnormal sugar tolerance are known to be seen. Using Zucker-lean rats to compare with the Zucker-fatty rats, the effect of *P. nebrodensis'* disease preventing and treating agents were studied on the rats.

### (1) Methods in Breeding the Test Rats and Administering the Test Substances

### [1] Preliminary Breeding (5 to 6 weeks old)

Five week old male Zucker-fatty rats and male Zucker-lean rats purchased from breeders were kept in animal chamber at a room temperature of 22±1°C and a relative humidity of 60±10%. The animals were exposed to light/dark cycles of 12 hours a day (lighting from 7:00 to 19:00) using a white fluorescence lamp and allowed to feed and drink freely for the 1 week of the preliminary breeding period.

After the preliminary breeding, individual animals were weighed and examined for glucose intolerance and the rats were split into groups of five, distributed so that the mean weight between the groups were as close as possible. The Zucker-fatty rats were split accordingly into five groups labeled K through O. The Zucker-lean rat groups were called groups P and Q.

### [2] P. nebrodensis Extract Preparation

The *P. nebrodensis* extract was prepared in the same manner as described in the previous experiment. (See Disease Preventing/Treating Agents Against Hypertension-Preparation of *P. nebrodensis* Extract)

### [3] Administration of Test Substances (6 to 16 weeks old)

Following the procedure stated below, the rats in each group were treated with test substances for 10 weeks, from the ages of 6 weeks to 16 weeks. The animals were allowed to feed and drink tap water freely. The animal chambers were kept at 22±1°C and 60±10% relative humidity with light/dark cycles using white fluorescence lamps for 12 hours a day (lighting from 7:00 to 19:00).

The animals were treated orally with each extract type at 10:00 am every day followed by sterilized water that was allotted freely.
Group K: Zucker-fatty Rats (no treatment)
Group L: Zucker-fatty Rats (3g Treatment Group)- 10 mL of the 3g extract per kg of body weight were administered daily
Group M: Zucker-fatty Rats (6g Treatment Group)- 10 mL of the 6g extract per kg of body weight were administered daily
Group N: Zucker-fatty Rats (9g Treatment Group)- 10 mL of the 9g extract per kg of body weight were administered daily
Group O: Zucker-fatty Rats (12g Treatment Group)- 10 mL of the 3g extract per kg of body weight were administered daily
Group P: Zucker-lean Rats (no treatment)
Group Q: Zucker-lean Rats (9g Treatment Group)- 10 mL of the 9g extract per kg of body weight were administered daily

### (2) Results

### [1] Weight Change

The animals were weighed once a week before treatment with the test substances. The mean values of the respective groups were obtained and are shown in Table 4.

**TABLE 4**

| Group | Zucker-fatty Rats | | | | | Zucker-lean Rats | |
|---|---|---|---|---|---|---|---|
| | K | L | M | N | O | P | Q |
| weight at 6 weeks of age (g) | 197 | 194 | 198 | 194 | 191 | 148 | 146 |
| weight at 8 weeks of age (g) | 321 | 342 | 308 | 312 | 322 | 239 | 233 |
| weight at 10 weeks of age (g) | 432 | 423 | 398 | 387 | 372 | 299 | 287 |
| weight at 12 weeks of age (g) | 503 | 491 | 454 | 436 | 433 | 357 | 347 |
| weight at 14 weeks of age (g) | 589 | 577 | 537 | 521 | 506 | 407 | 396 |
| weight at 16 weeks of age (g) | 641 | 631 | 598 | 584 | 571 | 443 | 423 |

The Zucker-fatty rats (Groups K through O) displayed a marked increase in body weight when compared with the Zucker-lean rats (Groups P and Q). However, the Zucker-fatty rats that had undergone treatment (Groups L through O) displayed an inhibition of weight gain at about 10 weeks from birth compared to the non-treated group (Group K). The inhibition of weight gain was more noticeable at higher doses.

Also in the treated Zucker-lean rat group (Group Q), weight gain inhibition was seen but the weight was still within the normal range.

Based on the findings above, the *P. nebrodensis* consistent to this invention exhibited a dose-dependent inhibitory effect on obesity and was proven to slow down fat deposition to a suitable degree, even in normal cases.

### [2] Blood Test

On the day prior to the final day of the experiment, all rats fasted. On the last day, the rats were deeply anesthetized with 45 mg/kg i.p. Nembutal and blood samples were taken at a large an amount as possible using a 20G needle at the left ventricle.

The blood was tested biochemical for certain components using an automatic chemical analyzer (Auto Lab, Radio Immuno Assay method). The group mean of each component were determined. The components and results are listed in Table 5.

**TABLE 5**

| Group | Zucker-fatty Rat | | | | | Zucker-lean Rat | |
|---|---|---|---|---|---|---|---|
| | K | L | M | N | O | P | Q |
| Total Cholesterol (mg/dL) | 155 | 145 | 136 | 119 | 109 | 77 | 71 |
| Free Cholesterol (mg/dL) | 40 | 35 | 29 | 25 | 27 | 29 | 28 |
| HDL Cholesterol (mg/dL) | 44 | 45 | 66 | 70 | 69 | 66 | 71 |
| LDL Cholesterol (mg/dL) | 7 | 6 | 5 | 6 | 6 | 5 | 6 |
| β Lipoprotein (mg/dL) | 702 | 643 | 443 | 345 | 587 | 143 | 132 |
| Triglycerides (mg/dL) | 667 | 561 | 327 | 344 | 310 | 67 | 63 |
| Free Fatty Acids (mg/dL) | 2.05 | 2.13 | 2.27 | 2.39 | 2.87 | 2.61 | 2.88 |
| Phospholipids (mg/dL) | 301 | 288 | 228 | 198 | 208 | 117 | 127 |
| Total Fat (g/dL) | 1245 | 1044 | 871 | 659 | 561 | 298 | 273 |
| Total Protein (g/dL) | 6 | 5.9 | 6.1 | 6.4 | 6.4 | 6.1 | 6.2 |
| Albumin (g/dL) | 3.7 | 3.4 | 3.4 | 3.9 | 3.8 | 3.8 | 3.7 |
| A/G | 1.62 | 1.67 | 1.66 | 1.65 | 1.64 | 1.67 | 1.67 |
| Urea Nitrogen (mg/dL) | 24 | 22 | 24 | 17 | 16 | 18 | 17 |
| Creatinine (mg/dL) | 0.4 | 0.5 | 0.7 | 0.9 | 0.9 | 0.8 | 0.9 |
| Uric Acid (mg/dL) | 1.9 | 2.2 | 2.8 | 2.7 | 3.4 | 3.4 | 3.6 |
| AST (GOT) (IU/I) | 766 | 651 | 445 | 423 | 434 | 409 | 443 |
| AST (GPT) (IU/I) | 299 | 256 | 198 | 208 | 187 | 198 | 174 |
| Insulin (mg/dL) | 26.9 | 23.8 | 22.8 | 23.6 | 18.9 | 1.2 | 1.1 |

As evident fro Table 5, the Zucker-fatty rats (Groups K to O) exhibited the higher levels of total cholesterol, free cholesterol, β lipoproteins, neutral fat, phospholipids, total lipids, urea nitrogen, AST (GOT), AST (GPT) and insulin compared to the Zucker-lean rats (Groups P and Q). The Zucker-fatty rats also showed lower levels of HDL cholesterol, free fatty acids, creatinine and uric acid than the Zucker-lean rats. Nevertheless, when compared to Group K (the group of Zucker-fatty rats that had no treatment) the treated groups, groups L through O exhibited a noticeable disease preventing/improving effect.

### Neutral Fat, Free Fatty Acids, Phospholipids and Total Lipids

When compared with Group K, levels of neutral fats, phospholipids and total lipids tend to decline and free fatty acids increase in groups L through O, all in a dose-dependent manner. Free fatty acids are attributed to neutral fat accumulated in adipose tissue that has subsequently been decomposed and released into the blood stream. Their presence in blood signifies that fat in the form of fatty acids are then utilized as an energy source in cardiac muscles, skeletal muscle, the kidneys and peripheral tissues. The finds above show that there is an improvement in fat loss indicated by the levels of free fatty acids. In addition, the reduction in phospholipids, which are necessary constituents in the cell membrane, was small in contrast with the noticeable reduction of neutral fat and total lipids. The level of phospholipids did not reach below the values in the healthy rats in group P. The effects described above were noted also in normal rats within a healthy range. Therefore, a beneficial effect on hyperlipidemia and obesity was seen.

### Cholesterol

As described before in the previous test, cholesterol values serve as an index of lipid dysbolism. When compared with the untreated group K, groups L through O displayed an increase in the good HDL cholesterol and there was a dose-dependent reduction in total and free cholesterol. Thus, the cholesterol levels revealed an improvement in lipid metabolism.

### β Lipoprotein

As indicated in the previous test, β lipoproteins serve as an index of lipid dysbolism.

Compared to group K, the treated groups L through O exhibited a reduction in the levels of β lipoprotein in a dose dependent manner. The levels came closer to that of group P, the normal rats. Lipid metabolism is seen so improve with treatment, as indicated by β lipoprotein levels.

### Insulin

Insulin is a hormone secreted by β cell in the pancreas. It stabilizes blood sugar levels by promoting glucose incorporation in muscle or fat tissue and inhibiting sugar release from the liver. It is also involved in glycogen accumulation, fat accumulation, ketone body reduction and protein synthesis. Insulin levels serve as an index of sugar dysbolism.

When compared with group K, groups treated with *P. nebrodensis,* groups L through O, displayed lower insulin levels close to that of the normal group (group P). As a result, along with reducing obesity, the *P. nebrodensis* agents have been seen to improve sugar metabolism.

### Urea Nitrogen, Creatinine, Uric Acid and AST

Levels of urea nitrogen, creatinine, uric acid and AST serve as indices of the hepatic and renal functions.

When compared with Group K, levels of the urea nitrogen and AST were reduced and levels of creatinine and uric acid were increased both in a dose-dependent manner in Groups L through O. The values where almost equal to the levels in the normal groups O and P, especially group O. In addition to reducing obesity, hepatic and renal functions have been seen to improve.

Total protein, albumin, A/G ratio, etc. which were normal initially, were seen not to be adversely affected by the treatment of extracts.

Based on the findings above, it has been seen that treatment with *P. nebrodensis* has beneficial effects on hyperlipidemia, obesity, lipid dysbolism, sugar metabolism, hepatic and renal functions.

### 3. Human Clinical Test

The clinical test procedures on the *P. nebrodensis* agents from this invention are discussed below.

### Preparation of Test Substance (Figure 1)

100 g of dried *P. nebrodensis* powder and 1 L of sterilized water were mixed in a conical flask and set in a water bath of 95°C for 1 hour and then allowed to cool to room temperature. The mixture was filtered under reduced pressure and the residue was separated from the filtrate. The residue was combined with 500mL of sterilized water and steeped again in a water bath of 95°C for 1 hour and cooled to room temperature. The mixture was filtered again and the first filtrate was combined with the second filtrate and concentrated under reduced pressure at 60°C. The mixture was sterilized by raising the temperature to 95°C for 10 minutes and re-concentrated at 60°C until it consisted of 50% solids. An excipient was added in amount of 50% based on solids and the mixture was dissolved through heating and lyophilized to obtain a powder. The powder was then sifted through a 60-mesh sieve and granulated using an extrusion granulation method.

Three males of 33, 42 and 50 years of age were treated orally with 6 g of the test substance daily and had blood samples taken and analyzed before treatment, at 1 month, 3 months and 6 months of treatment. The results are shown in Table 6.

**TABLE 6**

| | Before Treatment | 1 Month | 3 Months | 6 Months |
|---|---|---|---|---|
| Total cholesterol (mg/dL) | | | | |
| 33 yrs old | 287 | 283 | 224 | 217 |
| 42 yrs old | 276 | 257 | 234 | 207 |
| 50 yrs old | 296 | 291 | 247 | 210 |

| HDL Cholesterol (mg/dL) | | | | |
|---|---|---|---|---|
| 33 yrs old | 21 | 26 | 33 | 41 |
| 42 yrs old | 39 | 47 | 51 | 49 |
| 50 yrs old | 50 | 49 | 53 | 57 |

| Triglycerides (mg/dL) | | | | |
|---|---|---|---|---|
| 33 yrs old | 297 | 243 | 187 | 147 |
| 42 yrs old | 302 | 238 | 220 | 213 |
| 50 yrs old | 249 | 196 | 206 | 157 |

| β Lipoprotein (mg/dL) | | | | |
|---|---|---|---|---|
| 33 yrs old | 507 | 445 | 417 | 415 |
| 42 yrs old | 524 | 423 | 407 | 345 |
| 50 yrs old | 556 | 506 | 396 | 326 |

The normal values were reported to be 120 to 220 mg/dL of total cholesterol, 41 to 84 mg/dL of HDL cholesterol, 30 to 180 mg/dL of neutral fat, 150 to 500 mg/dL of the β lipoprotein. Before treatment all subjects had higher than normal levels of total cholesterol, neutral fat and β lipoprotein levels. All except one individual had HDL level lower than normal.

With treatment however, the levels improved and all three subjects recovered to the respective normal levels 6 months after the initial treatment. Thus *P. nebrodensis* was proven to exert disease preventing/treating effect in humans also. The treatment did not cause any side effects such as diarrhea, vomiting, abnormal urination, etc.

To summarize, following the incubation method in this present invention, sufficient yield of fruiting bodies of *P. nebrodensis* can be obtained within a short period by using electrical impulse and/or temperature change. Additionally, the agents in *P. nebrodensis* produced through this invention have been found to have beneficial disease preventing/treating effects.

## Claims

1. A method for incubating *Pleurotus nebrodensis* **characterized by** inoculation and cultivation of an inoculum of *P. nebrodensis* in which the temperature is maintained uniformly in the early-cultivating stage, decreased in the mid-cultivating stage and increased in the late-cultivating stage that allow the mycelium to proliferate and generate fruiting bodies.

2. A method for incubating *Pleurotus nebrodensis* **characterized by** an inoculation and cultivation of *P. nebrodensis* in a culture medium to allow mycelium proliferation and generation of fruiting bodies in which low temperature is maintained uniformly in the former-generating stage and increased in the latter-generating stage.

3. A method for incubating *Pleurotus nebrodensis* **characterized by** inoculating and cultivating *Pleurotus nebrodensis* in a culture medium that allows mycelium to proliferate over the culture medium and generate fruiting bodies in which the temperature is maintained uniformly in the early-cultivating stage, decreased in the mid-cultivating stage, sharply increased in the latter-generating stage and low temperature maintained in the former generating stage and increased in the latter generating stage.

4. A method for incubating *Pleurotus nebrodensis* using any method from claim 1 through 3 and in addition dispensing an electric impulse between 5 and 60 kV after the cultivation stage.

5. A method for incubating *Pleurotus nebrodensis* consistent with those of claim 1 through 3 wherein the temperature of the early cultivating stage is 16 to 24°C, the temperature of the mid-cultivating stage 6 to 14°C and the late cultivating stage 26 to 34°C.

6. A method for incubating *Pleurotus nebrodensis* consistent with claim 5 wherein the duration of the early-cultivation stage is 35 to 45 days, the mid-cultivation stage 5-15 days and the late-cultivation stage 5-15 days.

7. A method for incubating *Pleurotus nebrodensis* consistent to claim 1 or 3 in which the humidity is maintained at 65-75% in the cultivating stage.

8. A method for incubating *Pleurotus nebrodensis* in accordance to claim 2 or 3 wherein the temperature of the former generating stage is -5 to +3°C and the temperature of the latter-generating stage is 14 to 22°C.

9. A method for incubating *Pleurotus nebrodensis* according to claim 8 in which during the generating stage the temperature is increased by 2 steps.

10. A method for incubating *Pleurotus nebrodensis* according to claim 2 or 3, wherein in the generating stage, humidity is maintained between 75-85% and then increased to 90-100% at the same time the temperature is increased.

11. A method for incubating *Pleurotus nebrodensis* according to claim 2 or 3 wherein in the generating stage, the carbon dioxide level and/or illumination light intensity is increased at the same time as increasing temperature.

12. A method for incubating *Pleurotus nebrodensis* according to claim 2 or 3 in which during generation, the dead bacterial layer is removed before increasing temperature.

13. A method for incubating *Pleurotus nebrodensis* comprising of steps (a) to (d):
(a) A step for inoculating an inoculum *of Pleurotus nebrodensis* in a culture medium
(b) A step for an incubation at a temperature of 20-30°C whereby allowing the mycelium to proliferate over the culture medium after step (a)
(c) A step for giving an electric impulse at 5 to 60 kV after step (b); and,
(d) A step for generating fruiting bodies at a temperature of 10-20°C after step (c)

14. A method for incubating *Pleurotus nebrodensis* according to claim 13 in which the step (d), the temperature is temporarily decreased at -1 to 2°C and then increased at 10-20° C.

15. A disease preventing/treating agent which contains *P. nebrodensis* as a main ingredient.

16. A disease preventing/treating agent consistent to claim 15 comprised of a dried powder of *Pleurotus nebrodensis* and/or its hot water extract.

17. A disease preventing/treating agent according to claim 15 in which the disease is one or more of the following: hypertension, hyperlipidemia and obesity.
